# EUROPÄISCHE PATENTANMELDUNG

(11) **EP 1 798 213 A1**
(43) Veröffentlichungstag der Anmeldung: **20.06.2007**
(21) Anmeldenummer: 05027253.3
(22) Anmeldetag: 14.12.2005
(51) Int. Cl.: C07C 1/00

(54) **Verfahren zur Herstellung von Kohlenwasserstoffen**

(71) Anmelder: Cognis IP Management GmbH, 49589 Düsseldorf (DE)
(72) Erfinder: Falkowski, Jürgen, 40789 Monheim (DE); Dierker, Markus, Dr., 40597 Düsseldorf (DE); Neuss, Michael, Dr., 50997 Köln (DE); Schmid, Karl-Heinz, Dr., 40822 Mettmann (DE); Würkert, Stephan, 40223 Düsseldorf (DE)
(74) Vertreter: Reinhardt, Jürgen

(57) **Zusammenfassung**

Beschrieben wird ein Verfahren zur Herstellung von linearen gesättigten Alkanen aus primären Alkoholen deren C-Kette ein Kohlenstoffatom mehr enthält als das Alkan durch reduktive Dehydroxymethylierung der primären Alkohole in Gegenwart von Wasserstoff, einem Katalysator und bei Temperaturen von 100 bis 300 °C und Drücken von 1 bis 250 bar, wobei während der Reaktion entstehendes Wasser entfernt wird wobei als primäre Alkohole Fettalkohole mit 8 bis 24 Kohlenstoffatomen ausgewählt sind. Solche Alkane eignen sich zur Herstellung kosmetischer Mittel.

## Beschreibung

Die vorliegende Erfindung betrifft ein Verfahren zur Herstellung von Kohlenwasserstoffen aus Fettalkoholen und die Verwendung derartiger Kohlenwasserstoffen, vorzugsweise in kosmetischen Mitteln.

Leichtflüchtige Ölkörper, sogenannte light emollients', werden von der kosmetischen Industrie in einer Vielzahl von Formulierungen verwendet. Insbesondere für die dekorative Kosmetik bzw. in pflegenden Formulierungen werden grosse Mengen an leichterflüchtigen omponenten eingesetzt. Bei diesen Komponenten kann es sich beispielsweise um flüchtige, cyclische Silikone (z.B: Cyclopentasiloxan oder Cyclomethicon) oder Kohlenwasserstoffe aus petrochemischen Prozessen handeln. Bei den zuletzt genannten Stoffen handelt es sich aufgrund ihrer Herstellung überwiegend um Gemische aus linearen und verzweigten Kohlenwasserstoffen, deren Flammpunkt durchaus unter 50 °C (wie z.B. beim Isododecan) liegen kann. Beispiele und anwendungstechnische Beschreibungen derartiger Formulierungen können in Standardwerken, wie zum Beispiel: **,**Handbook of Cosmetic Science and Technology', A. Barel, M. Paye, H. Maibach, Marcel Dekker Inc. 2001 nachgelesen werden. Aus toxikologischen bzw. sicherheitstechnischen Gründen besteht jedoch in Zukunft Bedarf nach alternativen Rohstoffen für derartige Formulierungsaufgaben.

Die Aufgabe der Erfindung bestand darin, alternative Rohstoffe zu finden, die einerseits ökologisch bzw. toxikologisch unbedenklich sind und andererseits in typischen kosmetischen Formulierungen ohne anwendungstechnische Einschränkungen direkt ausgetauscht werden können.

Es wurde nun gefunden, dass über eine speziell geführte Reaktion Hydroxylverbindungen, wie z.B. Fettalkohole, mit hoher Selektivität in reine Kohlenwasserstoffe mit um ein C-Atom verkürzter Kettenlänge übergeführt werden können.

Ein erster Gegenstand der vorliegenden Erfindung betrifft daher ein Verfahren zur Herstellung von linearen gesättigten Alkanen aus primären Alkoholen deren C-Kette ein Kohlenstoffatom mehr enthält als das Alkan, durch Dehydroxymethylierung der primären Alkohole in Gegenwart von Wasserstoff, einem Katalysator und bei Temperaturen von 100 bis 300 °C und Drücken von 1 bis 250 bar und gleichzeitiger Wasserentfernung, wobei als primäre Alkohole Fettalkohole mit 8 bis 24 Kohlenstoffatomen ausgewählt sind.

Die hierbei ablaufende Reaktion wird als Dehydroxymethylierung oder auch reduktive Dehydroxymethylierung bezeichnet. Diese Reaktion ist für organische primäre Alkohole an sich bekannt. Aus dem Aufsatz **"**Hydrogenolysis of Alcohols"; Autoren: Hermann Pines und T.P. Kobylinski, Journal of Catalysis 17, 375-383 (1970) ist bekannt, dass Neopentylakohol u.a. zu Isobutan umgesetzt werden kann. Auch wird die Reaktion von Butanol zu Propan in Gegenwart von Nickel-Katalysatoren in einer Wasserstoffatmosphäre beschrieben. Es wird aber nicht die Verwendung langkettiger Fettalkohole für solche Reaktionen genannt. Die in diesem Aufsatz beschriebenen Reaktionen finden auch nicht in technischen Maßstab statt sondern wurden im Mikromaßstab durchgeführt. In dem Aufsatz **"**Direct Reduction of Alcohols to Hydrocarbons"; Autoren: W.F. Maier, I. This, P. Schleyer. Zeitschrift für Naturforschung, Teil B, 1982, 37B(3) wird zwar ebenfalls die reduktive Demethylierung primärer organischer Alkohole beschrieben, es werden aber keine langkettigen Fettalkohole als geeignete Edukte offenbart oder nahe gelegt. Die UK 1,051,826 beschreibt die reduktive Demethylierung von Diolen mit Nickel-Katalysatoren in einer Wasserstoffatmosphäre.

Aus den erfindungsgemäß erhaltenen Reaktionsgemischen können, vorzugsweise nach Aufreinigung der Rohprodukte, z.B. nach fraktionierter Destillation und weiterhin vorzugsweise nachfolgender Desodorierung, hochreine Kohlenwasserstoffe mit definierter Kettenlänge gewonnen werden. Die so erhaltenen Kohlenwasserstoffe definierter Kettenlänge können entweder als Einzelkomponenten in kosmetischen Formulierungen als sog. ,light emollients' verwendet werden oder definiert gemischt werden, um spezielle Eigenschaften, wie z.B. Spreitverhalten, Flüchtigkeit oder auch einen Flammpunkt einstellen zu können.

Die hydroxylhaltige Komponenten können Fettalkohole der oben angegebenen Kettenlänge sein, die wiederum nach einem dem Fachmann bekannten Verfahren aus nachwachsenden Rohstoffen wie z.B. Kokos-, Palm- oder Palmkernöl über Umesterung mit Methanol und anschließender Hydrierung hergestellt werden. Neben reinen Fettalkoholen können prinzipiell und bevorzugt auch andere, technisch hergestellte, lineare oder verzweigte, ein- oder mehrwertige Alkohole, Alkoholgemische oder derivatisierte Alkohole eingesetzt werden.

Besonders bevorzugt ist die Verwendung von Fettalkoholen mit geradzahligen C-Ketten, da so leicht die ansonsten schwer zugänglichen ungeradzahligen Alkane erhalten werden können. Bevorzugt werden als primäre Alkohole solche der allgemeinen Formel R-OH ausgewählt, in der R für einen gesättigten, linearen Alkylrest mit 8 bis 18, vorzugsweise 10 bis 16 und insbesondere 12 bis 16 Kohlenstoffatomen steht.

Die Reaktion der Alkohole zu den Kohlenwasserstoffen muss unter Zugabe von Wasserstoff und unter gleichzeitiger Wasserentfernung erfolgen.
Als Katalysatoren sind Platin Rubidium oder Nickel-haltige Katalysatoren besonders geeignet. Vorzugsweise werden aber Nickel-Katalysatoren verwendet und insbesondere handelsübliche Ni-haltige Hydrierkatalysatoren, wie z.B. Katalysatoren der Fa. Engelhard oder Kata Leuna. Die Katalysatoren können hierbei sowohl als Suspensionskatalysatoren für ein Semi-Batch - Verfahren, als auch als Festbettkatalysatoren für ein kontinuierliches Verfahren eingesetzt werden. Die Katalysatoren sind vorzugsweise im Mengen von 0, 1 bis 3 Gew.-%, bezogen auf die Menge an primären Fettalkoholen in der Reaktionsmischung vorhanden. Bevorzugt können auch Katalysatoren in Mengen von 0,2 bis 2 Gew.-% und insbesondere in Mengen von 0,5 bis 1,0 Gew.-% Verwendung finden. Bei einem Suspensionsverfahren hat sich eine Katalysatorkonzentration von 0,1 - 2 Gew.-% ig bezüglich auf die eingesetzte Menge an Fettalkohol als geeignet erwiesen, wobei der bevorzugte Bereich bei 0,5 - 1,0 Gew.-% Ni liegt.

Die für das Verfahren üblicherweise erforderliche Reaktionstemperatur beträgt 180 °C - 300°C, wobei der bevorzugte Bereich bei 200 - 280 °C liegt und insbesondere bevorzugt bei 220°C - 260 °C liegt.
Der für das Verfahren üblicherweise geeignete Reaktionsdruck beträgt 0 - 250 bar, wobei 1 bis 100 bar bevorzugt ist. Besonders bevorzugt ist der Bereich bei 5 - 80 bar und insbesondere von 10-50 bar.

Während der Reaktion ist es entstehendes Reaktionswasser zu entfernen. Daher hat es sich als vorteilhaft erwiesen, Wasserstoff zu dem mit suspendiertem Katalysator vorgelegtem Alkohol zu dosieren und gleichzeitig entstehendes Reaktionswasser bzw. Reaktionsgase aus dem Reaktor zu entfernen. Bei einem kontinuierlichen Verfahren kann diese Wasserauskreisauskreisung beispielsweise in einem mehrstufigen Verfahren erfolgen. Das entstandene Reaktionsgemisch muss anschließend zur Katalysatorabtrennung filtriert werden.

Danach wird zur Entfernung des Restalkoholgehaltes bzw. von Spuren dimerer Reaktionsprodukte eine fraktionierte Destillation durchgeführt. Das hierbei erhaltene Sumpfprodukt kann für den nächsten Ansatz recyclisiert werden.
Anschließend kann zur Geruchsverbesserung noch eine Desodorierung nachgeschaltet werden.

In einer weiteren Ausführungsform betrifft die vorliegende Erfindung die Verwendung der gemäß dem obigen Verfahren hergestellten Kohlenwasserstoffe in kosmetischen Mitteln. Die erfindungsgemäß hergestellten Kohlenwasserstoffe können zur Herstellung von kosmetischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmit-tel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Bevorzugt ist die Verwendung als Ölkörper.

Mit der hier getätigten Erfindung besteht gezielt die Möglichkeit, Kohlenwasserstoffe definierter Kettenlänge als Einzelkomponenten in kosmetischen Formulierungen als sog. 'light emollients' zu verwenden oder sogar definiert zu mischen, um spezielle Eigenschaften, wie z.B. Spreitverhalten, Flüchtigkeit oder auch Flammpunkte einstellen zu können. Insbesondere durch die Möglichkeit einer Mischung dieser Kohlenwasserstoffe nach dem Baukastenprinzip ergeben sich somit wesentliche Vorteile gegenüber Kohlenwasserstoffen aus petrochemischen Quellen, die fast ausschließlich als komplexe Gemische aus verzweigten und unverzweigten Kohlenwasserstoffen vorliegen. Eine weitere destillative Aufarbeitung ist in diesen Fällen nur mit hohem Aufwand möglich, bzw. hätten das Problem, das Restmengen an unverwünschten Isomeren im Produkt verbleiben würden. Darüber hinaus ist - eine insbesondere für kosmetische Anwendungen wichtige - toxikologische Bewertung eines definierten Kohlenwasserstoffes bzw. einer definierten Kohlenwasserstoffmischung sehr viel einfacher und sicherer.

### 1) Herstellung von Tridecan aus 1-Tetradecanol

1000 g 1-Tetradecanol (4,7 mol; Lorol C 14 der Fa. Cognis) wurden in einem rührbaren Druckbehälter mit 10 g eines Nickelkatalysators (Ni-5249 P der Fa. Engelhard; Ni-Gewalt = 63 Gew.-%) vorgelegt und auf 240 °C aufgeheizt. Anschließend wurdu über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Es ergab sich eine Auswaage von 845 g Reaktionsprodukt.

| | |
|---|---|
| Eine GC-Analyse ergibt folgende Zusammensetzung: | 89,0 % Tridecan |
| | 2,1 % Tetradecan |
| | 4,1 % 1-Tetradecanol |
| | 4,2 % Dimere Reaktionsprodukte |

Dieses Reaktionsprodukt wird danach in einer Destillation zum reinem Tridecan fraktioniert und anschließend mit Stickstoff desodoriert. Man erhält ein farbloses, dünnflüssiges und geruchsarmes Produkt.

### 2) Herstellung von Undecan aus 1-Dodecanol

1000 g 1-Dodecanol (5,4 mol; Lorol C 12 der Fa. Cognis) wurden in einem rührbaren Druckbehälter mit 10 g eines Nickelkatalysators (Ni-5249 P der Fa. Engelhard; Ni-Gewalt = 63 Gew%) vorgelegt und auf 240 °C aufgeheizt. Anschließend wurde über einen Zeitraum von 8 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt und abgelassen und filtriert. Es ergab sich eine Auswaage von 835g Reaktionsprodukt.

| | |
|---|---|
| Eine GC-Analyse ergibt folgende Zusammensetzung: | 68,4 % Undecan |
| | 0,6 % Dodecan |
| | 21,7 % 1-Dodecanol |
| | 7,2 % Dimere Reaktionsprodukte |

Dieses Reaktionsprodukt wurde danach Destilliert, um das Undecan rein zu erhalten. Dieses wurde anschließend mit Stickstoff desodoriert. Man erhält ein farbloses, dünnflüssiges und geruchsarmes Produkt.

## Patentansprüche

1. Verfahren zur Herstellung von linearen gesättigten Alkanen aus primären Alkoholen deren C-Kette ein Kohlenstoffatom mehr enthält als das Alkan durch reduktive Dehydroxymethylierung der primären Alkohole in Gegenwart von Wasserstoff, einem Katalysator und bei Temperaturen von 100 bis 300 °C und Drücken von 1 bis 250 bar, wobei während der Reaktion entstehendes Wasser entfernt wird, **dadurch gekennzeichnet, dass** als primäre Alkohole Fettalkohole mit 8 bis 24 Kohlenstoffatomen ausgewählt sind.

2. Verfahren nach Anspruch 1, **dadurch gekennzeichnet, dass** die Reaktion bei Temperaturen von 180 bis 300 °C, vorzugsweise 200 bis 280 °C und insbesondere von 220 bis 260 °C durchgeführt wird.

3. Verfahren nach den Ansprüchen 1 bis 2, **dadurch gekennzeichnet, dass** das Verfahren bei Drücken von 1 bis 100 bar, vorzugsweise 5 bis 80 bar und insbesondere von 10 bis 50 bar durchgeführt wird.

4. Verfahren nach den Ansprüchen 1 bis 3, **dadurch gekennzeichnet, dass** als Katalysatoren solche ausgewählt sind, die Nickel enthalten.

5. Verfahren nach den Ansprüchen 1 bis 4, **dadurch gekennzeichnet, dass** der Katalysator in Mengen von 0,1 bis 3 Gew.-% vorzugsweise von 0,2 bis 2 Gew.-% und insbesondere in Mengen von 0,5 bis 1,0 Gew.-%, jeweils bezogen auf die Menge an eingesetztem Fettalkohol vorliegt.

6. Verfahren nach den Ansprüchen 1 bis 5, **dadurch gekennzeichnet, dass** als primäre Alkohole solche der allgemeinen Formel R-OH ausgewählt sind, in der R für einen gesättigten, linearen Alkylrest mit 8 bis 18, vorzugsweise 10 bis 16 und insbesondere 12 bis 16 Kohlenstoffatomen steht.

7. Verfahren nach einem der Ansprüche 1 bis 7, **dadurch gekennzeichnet, dass** die primären Alkohole eine geradzahlige Kohlenstoffanzahl enthalten.

8. Verfahren nach einem der Ansprüche 1 bis 8, **dadurch gekennzeichnet, dass** Mischungen der primären Alkohole eingesetzt werden.

9. Verfahren nach einem der Ansprüche 1 bis 9, **dadurch gekennzeichnet, dass** im Anschluss an die Reaktion das erhaltene Produkt gereinigt wird.

10. Verwendung von Fettalkoholen, die gemäß dem Verfahren nach Anspruch 1 hergestellt worden sind in kosmetischen Mitteln.
